# EUROPÄISCHE PATENTANMELDUNG

(11) **EP 2 289 506 A2**
(43) Veröffentlichungstag der Anmeldung: **02.03.2011**
(21) Anmeldenummer: 10009997.7
(22) Anmeldetag: 18.11.2002
(51) Int. Cl.: A61K 31/23, A61K 31/25, A61K 31/353, A61P 17/00, A61P 31/20, A61P 31/22

(54) **Arzneimittel enthaltend eine C11-C21 Alkyl-, Alkenyl- oder Alkinylsäureester und ein Catechin**

(30) Priorität: 19.11.2001 DE 10156794; 19.11.2001 US 331705 P
(62) Teilanmeldung aus: 08021728.4
(71) Anmelder: MediGene AG, 82152 Martinsried (DE)
(72) Erfinder: Chang, Yunik, Sonoma, CA 95476 (US); Lathrop, Robert, Fort Collins, CA 80526 (US); Böhm, Erwin, 68526 Ladenburg (DE); Gander-Meisterernst, Irene, 48149 Münster (DE); Greger, Regina, 52393 Iffeldorf (DE); Holldack, Johanna, 10785 Ebeltoft (DK); Mebius, Ulrich, 82131 Gauting-Unterbrunn (DE)
(74) Vertreter: Bösl, Raphael Konrad

(57) **Zusammenfassung**

Die vorliegende Erfindung betrifft ein Arzneimittel, enthaltend eine Verbindung der allgemeinen Formel 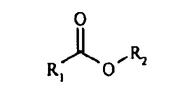 wobei, R₁ unabhängig voneinander ein unverzweigter oder verzweigter, gesättigter, einfach oder mehrfach ungesättigter, gegebenenfalls substituierter C₁₁-C₂₁ Alkyl-, Alkylen- oder Alkinylrest, vorzugsweise ein C₁₁-C₁₅- Alkyl-, Alkylen- oder Alkinylrest, insbesondere ein C₁₁-C₁₃ Alkyl-, Alkylen- oder Alkinylrest, vor allem ein C₁₃-Alkylrest bedeutet, und
R₂ unabhängig voneinander ein unverzweigter oder verzweigter C₁-C₈ Alkyl Alkylen- oder Alkinylrest, vorzugsweise ein C₁-C₆- Alkyl-, Alkylen- oder Alkinylrest, insbesondere ein C₂-C₄ Alkyl-, Alkylen- oder Alkinylrest, vor allem ein C₃-Alkylrest, ein -[CH₂-(CH₂)ₘ-O]ₙ-H Rest mit n = 1 bis 10, vorzugsweise n = 1 bis 5, m = 1 bis 5, vorzugsweise m = 1 bis 3,
einen -CH₂-[CH-(OH)]ₚ-[CH₂-(R₃)]-Rest, wobei R₃ unabhängig voneinander ein Wasserstoff oder ein Hydroxylrest, p = 1 bis 7, vorzugsweise p = 1 bis 4, ein Pentose-Rest oder ein Hexose-Rest bedeutet,
als therapeutisch aktiven Wirkstoff alleine oder zusammen mit einem oder mehreren weiteren pharmazeutischen Wirkstoffen als Kombinationspräparat zur Behandlung von viralen Hauterkrankungen und/oder Tumorerkrankungen, hervorgerufen insbesondere durch humane Papilloma Viren (HPV) und/oder Herpes Viren, sowie eine topisch wirkende Arzneimittelformulierung und deren Verwendung.

## Beschreibung

Die vorliegende Erfindung betrifft ein Arzneimittel, enthaltend eine Verbindung der allgemeinen Formel 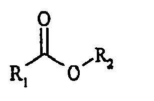 wobei, R₁ unabhängig voneinander ein unverzweigter oder verzweigter, gesättigter, einfach oder mehrfach ungesättigter, gegebenenfalls substituierter C₁₁-C₂₁ Alkyl-, Alkylen- oder Alkinylrest, vorzugsweise ein C₁₁-C₁₅- Alkyl-, Alkylen- oder Alkinylrest, insbesondere ein C₁₁-C₁₃ Alkyl-, Alkylen- oder Alkinylrest, vor allem ein C₁₃-Alkylrest bedeutet, und
R₂ unabhängig voneinander ein unverzweigter oder verzweigter C₁-C₈ Alkyl Alkylen- oder Alkinylrest, vorzugsweise ein C₁-C₆- Alkyl-, Alkylen- oder Alkinylrest, insbesondere ein C₂-C₄ Alkyl-, Alkylen- oder Alkinylrest, vor allem ein C₃-Alkylrest, ein -[CH₂-(CH₂)ₘ-O]ₙ-H Rest mit n = 1 bis 10, vorzugsweise n = 1 bis 5, m = 1 bis 5, vorzugsweise m = 1 bis 3,
einen -CH₂-[CH-(OH)]ₚ-[CH₂-(R₃)]-Rest, wobei R₃ unabhängig voneinander ein Wasserstoff oder ein Hydroxylrest, p = 1 bis 7, vorzugsweise p = 1 bis 4, ein Pentose-Rest oder ein Hexose-Rest bedeutet,
als therapeutisch aktiven Wirkstoff alleine oder zusammen mit einem oder mehreren weiteren pharmazeutischen Wirkstoffen als Kombinationspräparat zur Behandlung von viralen Hauterkrankungen und/oder Tumorerkrankungen, hervorgerufen insbesondere durch humane Papilloma Viren (HPV) und/oder Herpes Viren, sowie eine topisch wirkende Arzneimittelformulierung und deren Verwendung.

Papilloma Viren (HPV) sind DNA-Viren, die die Epithelialzellen von Säugetieren infizieren und dadurch ein unkontrolliertes Zellwachstum hervorrufen. Es gibt die unterschiedlichsten Arten von Papilloma Viren, die Menschen und verschiedene Tierarten infizieren. Alle Arten infizieren hierbei die basalen Epithelialzellen und verbleiben episomal oder integrieren ihre DNA ins Wirtsgenom.

Es ist seit langem bekannt, dass Papilloma Viren Genitalwarzen (Condyloma acuminata), gewöhnliche und plantare Warzen, bowenoide Papulose bei Männern und Frauen, und cervicale intra-epitheliale Neoplasien bei Frauen hervorrufen.

Je nach Methode liegt die Detektionsrate der HPV bei fast 100 %. In Warzen und Genitalwarzen (Condyloma acuminata)werden vor allem HPV 6 und HPV 11 Viren gefunden. Da HPV 16 und HPV 18 vor allem in malignen, schuppigen Zellkarzinomen wie bei Penis- und Gebärmutterhalskrebs beobachtet wird, ist allgemein akzeptiert, dass HPV 16 und HPV 18 in Verbindung mit bösartigen HPV-Erkrankungen steht.

Durch humane Papilloma Viren verursachte Genitalwarzen werden gegenwärtig überwiegend mit physikalischen Methoden behandelt. Hierzu gehören die operative Entfernung, Elektrokauterisierung, Kryochirurgie oder Lasertherapie, um nur einige zu nennen. Eine weitere medizinische Behandlung ist die Anwendung von Podophyllin, 5-Fluoruracil, Bleomycin, Interferon, Imiquimod, usw.

Die operative Behandlung hat den Nachteil, dass sie für den Patienten sehr unangenehm ist und zu weiteren Infektionen führen kann. Bei einer topischen Anwendung bestand bisher das Risiko von Nebenwirkungen, da die verwendeten Wirkstoffe cytotoxische Eigenschaften besitzen oder die zellulare Immunabwehr verstärken und somit lokale Entzündungen hervorrufen können. Dies zeigt, dass die bisher vorhandenen Therapiemöglichkeiten noch nicht zufriedenstellend sind.

Hinzu kommt, dass die Rückfallquote bei Warzen und Genitalwarzen sehr hoch ist und eine komplette Heilung nur durch konstante und konsequente Behandlung möglich ist. Aus diesem Grund besteht der Wunsch nach einer sicheren und bequemen Behandlung.

Besonders bei der Behandlung von Gentialwarzen, aber auch bei allen übrigen Krankheiten, die durch das Papilloma Virus hervorgerufen werden, ist eine Behandlung erwünscht, die für den Patienten leicht anwendbar ist. Geeignet wäre beispielweise eine Behandlung die der Patient selbst bei den betroffenen Stellen anwenden kann und die in relativ kurzer Zeit gute Resultate zeigt und nur geringe bzw. keine Nebenwirkungen zeigt.

Herpes Simplex Viren (HSV) sind DNA-Viren aus der Alphasubfamilie Herpetoviridae. Sie sind in zwei Gruppen eingeteilt, den HSV 1, dem sogenannten oralen Stamm und den HSV 2, dem sogenannten genitalen Stamm. Herpes Simplex Viren penetrieren als Nukleokapsid in die Nervenendigungen und gelangen mit dem axonalen Strom in die zugehörigen Ganglien. Sie werden durch Schmier- und Tröpfcheninfektion aus Herpesläsionen oder von gesunden Dauerausscheidern übertragen. Nach einer Primärinfektion kann es zu einer erneuten, symptomatischen oder asymptomatischen Reaktivierung der Viren durch eine Irritation, z. B. durch Fieber, Traumata oder Strahlung, von latent infizierten Neuronen kommen. Diese Reaktivierung hängt insbesondere von der Abwehrlage des gesamtem Organismus ab. Herpes Simples Viren können Zellen in Tieren und Zellkulturen neoplastisch transformieren. Es wird derzeit ein Zusammenspiel von Herpes Simplex Viren vom Typ 2 und der Genese von Zervixkarzinomen mit den humanen Papilloma Viren vom Typ 16 und 18 diskutiert.

Eine Behandlung von Herpesinfektionen durch Behandlung mit einer Mischung aus Isopropylmyristat und kleinen Mengen von 4-{Niedrigalkyl}-2,6-(bis-*tert-*Butyl)-Phenol mit 2,6-(bis-*tert*-Butyl)-4-Hydroxy-Toluol bzw. 4-(Lliedrigalkyl)-2,6-(bis-*tert*-Butyl)-Phenol ist bereits aus der EP 0 087 161 bekannt.

In der EP 0 842 660 ist eine pharmazeutische Zusammensetzung zur Behandlung von Genitalwarzen, die durch humane Papilloma Viren verursacht werden, beschrieben. Die beschriebene Zusammensetzung enthält Catechine aus Tee-Extrakten (Camellia sinensis), überwiegend (-)-Epigallocatechin-gallat in Form einer Salbe oder eines Zäpfchens.

Aufgabe der vorliegenden Erfindung ist daher, weitere wirksame antivirale Substanzen und Formulierungen zu finden, die zur Behandlung von viralen Hauterkrankungen und/oder Tumorerkrankungen, hervorgerufen durch Papilloma Viren und/oder Herpes Viren, geeignet sind.

Überraschenderweise wurde nun gefunden, dass ein Arzneimittel, enthaltend eine anspruchsgemäße Verbindung der allgemeinen Formel (I) als pharmazeutisch aktiven Wirkstoff, dazu geeignet ist, virale Hauterkrankungen und/oder Tumorerkrankungen zu behandeln.

Die vorliegende Erfindung betrifft somit ein Arzneimittel enthaltend eine Verbindung der allgemeine Formel (I) als pharmazeutisch aktiven Wirkstoff,

A-B (I)

wobei A ein Rest der allgemeinen Formel (II) 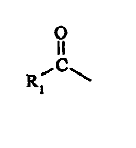 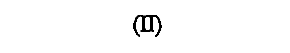 und B ein Rest der allgemeinen Formel (III)

-O-R₂ (III)

bedeutet, und
R₁ unabhängig voneinander ein unverzweigter oder verzweigter, gesättigter, einfach oder mehrfach ungesättigter, gegebenenfalls substituierter C₁₁-C₂₁ Alkyl-, Alkylen- oder Alkinylrest, vorzugsweise ein C₁₁-C₁₅- Alkyl-, Alkylen- oder Alkinylrest, insbesondere ein C₁₁-C₁₃ Alkyl-, Alkylen- oder Alkinylrest, vor allem ein C₁₃-Alkylrest bedeutet, und
R₂ unabhängig voneinander ein unverzweigter oder verzweigter C₁-C₈ Alkyl Alkylen- oder Alkinylrest, vorzugsweise ein C₁-C₆- Alkyl-, Alkylen- oder Alkinylrest, insbesondere ein C₂-C₄ Alkyl-, Alkylen- oder Alkinylrest, vor allem ein C₃-Alkylrest, ein -[CH₂-(CH₂)ₘ-O]ₙ-H Rest mit n = 1 bis 10, vorzugsweise n = 1 bis 5, m = 1 bis 5, vorzugsweise m = 1 bis 3,
einen -CH₂-[CH-(OH)]ₚ-[CH₂-(R₃)]-Rest, wobei R₃ unabhängig voneinander ein Wasserstoff oder ein Hydroxylrest, p = 1 bis 7, vorzugsweise p = 1 bis 4, ein Pentose-Rest oder ein Hexose-Rest bedeutet

Der Rest R₁ und oder der Rest R₂ können dabei unabhängig voneinander mit einem Halogen, vorzugsweise Fluor und/oder Chlor, einem unverzweigten oder verzweigten C₁-C₆- Alkyl-, Alkylen- oder Alkinylrest, vorzugsweise einem C₁-C₃-Alkyl-, Alkylen- oder Alkinylrest, insbesondere einem Methylrest substituiert sind.

Der Rest A der Verbindung (I) kann dabei beispielsweise abgeleitet sein von Hexansäure (Capronsäure), Heptansäure, Octansäure (Caprylsäure), Nonansäure, Decansäure (Caprinsäure), Undecansäure, Dodecansäure (Laurinsäure), Tridecansäure, Tetradecansäure (Myristinsäure), Pentadecansäure, Hexadecansäure (Palmitinsäure), Heptadecansäure, Octadecansäure (Stearinsäure), Nonadecansäure, Eicosansäure, Heneicosansäure, Ölsäure, Linolsäure, Linolensäure und/oder Arachidonsäure, bevorzugt von Capronsäure, Caprylsäure, Caprinsäure, Laurinsäure, Myristinsäure, Palmitinsäure, Stearinsäure, Ölsäure, Linolsäure, Linolensäure und/oder Arachidonsäure, besonders bevorzugt von Myristinsäure.

Der Rest B der Verbindung (I) kann beispielsweise abgeleitet sein von einem unverzweigten oder verzweigten C₁-C₈ Alkylalkohol, insbesondere Ethanol, Propanol, Isopropanol, n-Butanol, *tert*-Butanol besonders bevorzugt Isopropanol, Ethylenglykol, Polyethylenglykol, Propylenglykol, Polypropylenglykol, Glycerin, Polyglycerin, einem Pentose-Zucker wie beispielsweise Arabit, Adonit und Xylit, oder einem Hexose-Zucker wie beipsielsweise Sorbit, Mannit oder Dulcit, bevorzugt von Ethanol, Propanol, Isopropanol, Butanol, Ethylenglykol, Polyethylenglykol, Propylenglykol, Polyproylenglykol, Glycerin, Polyglycerin, Arabit, Adonit, Xylit, Sorbit, Mannit, und/oder Dulcit.

Die Verbindung (I) ist bevorzugt Isopropyllaureat, Isopropylmyristat, Isopropylpalmitat, Isopropylstearat, Ethylmyristat, Propylmyristat, Butylmyristat und/oder Ethyloleat, insbesondere Isopropylmyristat.

In einer weiteren bevorzugten Ausführungsform ist die Verbindung (I) eine hydrophobe Verbindung. Unter einer hydrophoben Verbindung wird gemäß der vorliegenden Erfindung, eine Verbindung verstanden, deren Löslichkeit in Wasser maximal ca. 0,2 mg/ml, insbesondere maximal ca. 0,1 mg/ml beträgt

Das Arzneimittel enthält beispielsweise mindestens ca. 5 % - 75 % (w/w), vorzugsweise mindestens ca. 10 % - 60 % (w/w), insbesondere mindestens ca. 25 % - 55 % (w/w), und vor allem mindestens ca. 35 % - 50 % (w/w) der Verbindung der allgemeinen Formel (I).

Das erfindungsgemäße Arzneimittel kann hierbei zusätzlich einen oder mehrere weitere pharmazeutische Wirkstoffe als Kombinationspräparat zur gleichzeitigen, getrennten oder zeitlich abgestuften Anwendung enthalten. Als weitere pharmazeutische Wirkstoffe werden hier bevorzugt solche eingesetzt, die zur Behandlung von viralen Hauterkrankungen und/oder Tumorerkrankungen verwendet werden können, wie beispielsweise Podophyllin, 5-Fluoruracil, Bleomycin, Interferon, Imiquimod, und/oder Gemische enthaltend mindestens ein Catechin.

In einer bevorzugten Ausführungsform ist der weitere pharmazeutische Wirkstoff ein amphiphiler bzw. amphipathischer Wirkstoff. Unter einem amphiphiler bzw. amphipathischer Wirkstoff wird ein Wirkstoff verstanden, der aus zwei funktionellen Teilen aufgebaut ist, insbesondere einem hydrophilen und einem lipophilen Teil. Durch diese Eigenschaft könnte im Besonderen der Durchtritt der Substanz durch die Haut erleichtert und eine bessere Wirkung erzielt werden. Die bessere Wirkung kann dabei beispielsweise auf eine längere Verweildauer am gewünschten Ort oder auf eine Dosisreduktion des Wirkstoffes zurückzuführen sein.

In einer weiteren bevorzugten Ausführungsform enthält der weitere pharmazeutische Wirkstoff mindestens ein Catechin der allgemeinen Formel (IV) 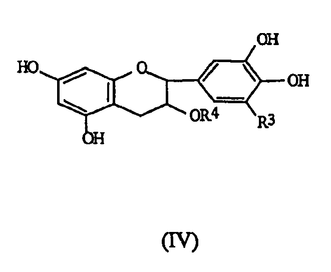 worin,
R₃ ein -H oder -OH und
R₄ ein -H oder eine Gruppe der Formel (V) 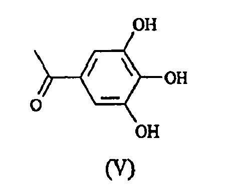 bedeutet.

Die hierbei zugesetzten Catechine können entweder synthetisch oder aus natürlichen Quellen gewonnen werden. Als natürliche Quellen sind vor allem Teesträucher zu nennen. Je nach Art und Sorte können hier die natürlichen Bestandteile in unterschiedlichen Konzentrationen vorhanden sein. Hierbei werden die verwendeten Catechine bevorzugt aus Camellia sinensis, Camellia asamica, Camellia bohea, Camellia chinensis oder Camellia oleosa gewonnen. Zur Gewinnung der Catechine können sämtliche Bestandteile von Teesträuchern, insbesondere die Blätter, verwendet werden. Bevorzugt werden die verwendeten Catechine aus einem Teeextrakt gewonnen.

Die verwendeten Catechine der vorliegenden Erfindung sind bevorzugt Epicatechin, Epicatechin Gallat, Epigallocatechin, Epigallocatechin Gallat, Gallocatechin und Gallocatechin Gallat, insbesondere (-)-Epicatechin, (-)-Epicatechin Gallat, (-)-Epigallocatechin, (-)-Epigallocatechin Gallat, (+)-Gallocatechin und (-)-Gallocatechin Gallat.

Die Catechine können sowohl einzeln, als auch in Form von Gemischen mit unterschiedlichen Zusammensetzungen verwendet werden. Ein Catechingemisch enthält ca. 2-20 % (w/w), vorzugsweise ca. 4-15 % (w/w), insbesondere ca. 10-11 % (w/w) (-)-Epicatechin, ca. 2-20 % (w/w), vorzugsweise ca. 5-15 % (w/w), insbesondere ca. 5-7% (w/w) (-)-Epicatechin Gallat, ca. 1-25 % (w/w), vorzugsweise ca. 3-15% (w/w), insbesondere ca. 5-7 % (w/w) (-)-Epigallocatechin, ca. 40-75 % (w/w), vorzugsweise ca. 57-67 % (w/w), insbesondere ca. 61-66 % (w/w) (-)-Epigallocatechin Gallat, ca. 0,05-5 % (w/w), vorzugsweise ca. 0,1-1 % (w/w), insbesondere ca. 0,1-0,6 % (w/w) (+)-Gallocatechin und/oder ca. 0,5-20 % (w/w), vorzugsweise ca. 1-10 % (w/w), insbesondere ca.1-5 % (w/w) (-)-Gallocatechin Gallat.

In einer bevorzugten Ausführungsform besteht das Catechingemisch aus ca. 5,9 % (w/w) (-)-Epicatechin, ca. 12,6 % (w/w) (-)-Epicatechin Gallat, ca. 17,6 % (w/w) (-)-Epigallocatechin, ca. 53,9 % (w/w) (-)-Epigallocatechin Gallat und/oder ca. 1,4 % (w/w) (-)-Gallocatechin. Eine derartige Zusammensetzung ist unter dem Namen Polyphenon®100 bekannt.

In einer besonders bevorzugten Ausführungsform besteht das Catechingemisch aus ca. 10,8 % (w/w) (-)-Epicatechin, ca. 6,5 % (w/w) (-)-Epicatechin Gallat, ca. 9,2 % (w/w) (-)-Epigallocatechin, ca. 54,8 % (w/w) (-)-Epigallocatechin Gallat und/oder ca. 4,0 % (w/w) (-)-Gallocatechin Gallat. Eine derartige Zusammensetzung ist unter dem Namen Polyphenon® E bekannt.

Das erfindungsgemäße Arzneimittel enthält beispielsweise ca. 1-30 % (w/w), vorzugsweise ca. 2-20 % (w/w) und insbesondere ca. 15-18 % (w/w) eines Catechins sowie mindestens ca. 5-90 % (w/w), vorzugsweise mindestens ca. 10-70 % (w/w), insbesondere mindestens ca. 25-60 % (w/w) und vor allem mindestens ca. 35-50 % (w/w) der Verbindung (I).

Die Herstellung von Arzneimitteln mit einem Gehalt an einer oder mehreren erfindungsgemäßen Verbindungen bzw. deren Einsatz bei der erfindungsgemäßen Verwendung erfolgt in üblicher Weise anhand geläufiger pharmazeutischtechnologischer Verfahren. Dazu werden die Wirkstoffe zusammen mit geeigneten, pharmazeutisch annehmbaren Hilfs- und Trägerstoffen zu den für die verschiedenen Indikationen und Applikationsorte geeigneten Arzneiformen verarbeitet. Dabei können die Arzneimittel in der Weise hergestellt werden, dass die jeweils erwünschte Freisetzungsrate, z.B. eine rasche Anflutung und/oder ein Retard- bzw. Depoteffekt, erzielt wird.

Zur herkömmlichen Applikation auf der Haut oder Schleimhaut lassen sich übliche Emulsionen, Gele, Salben, Cremes der mischphasigen bzw. amphiphilen Emulsionssysteme (Öl/Wasser-Wasser/Öl-Nfischphase) sowie Liposomen und Transfersomen oder Pflaster, bevorzugt Salben und Cremes, besonders bevorzugt eine Salbe anführen. Vorzugsweise wird der Wirkstoff lokal in dem Bereich appliziert, in dem eine Haut- bzw. Schleimhautveränderung und/oder -erkrankung vorliegt.

Als topisch, lokal oder regional verabreichbare Arzneimittel kommen neben den bekannten Anwendungen auf der Haut und/oder Schleimhaut als spezielle Zubereitungen die folgenden in Betracht: genital, vaginal oder rektal, insbesondere genital und vaginal applizierbare Emulsionen, Cremes, Salben, Schaumtabletten oder Suppositorien. Auf der Grundlage von Gelatine oder anderen Trägerstoffen lassen sich auch Rektalkapseln bereitstellen. Als Suppositoriengrundlagen kommen Hartfette, wie z.B. Witepsol®, Massa Estarium®, Novata®, Kokosfett, Glycerin/Gelatine-Massen, Glycerin/Seifen-Gele und Polyethylenglykole in Betracht.

Als Hilfs- bzw. Trägerstoffe eignen sich beispielsweise Natriumalginat als Gel-bildner zur Herstellung einer geeigneten Grundlage oder Cellulosederivate, wie z. B. Guar- oder Xanthangummi, anorganische Gelbildner, wie z.B. Aluminiumhydroxide oder Bentonite (sog. thixotrope Gelbildner), Polyacrylsäurederivate, wie z.B. Carbopol®, Polyvinylpyrrolidon, mikrokristalline Cellulose oder Carboxymethylcellulose. Weiterhin kommen amphiphile nieder- und höhermolekulare Verbindungen sowie Phospholipide in Betracht. Die Gele können entweder als Hydrogele auf Wasserbasis oder als hydrophobe Organogele, beispielsweise auf Basis von Gemischen nieder- und hochmolekularer Paraffinkohlenwasserstoffe und Vaseline vorliegen. Die hydrophilen Organogele können beispielsweise auf Basis hochmolekularer Polyethylenglykole zubereitet werden. Diese gelartigen Formen sind abwaschbar. Unter den Organogelen sind die hydrophoben Organogele jedoch bevorzugt. Besonders bevorzugt sind hydrophobe Hilfsstoffe- und Zusatzstoffe wie Petrolatum, Wachs, Oleylalkohol, Propylenglykolmonostearat und Propylenglykolmonopalmitostearat. Dem Fachmann bekannte hautberuhigende und/oder endzündungshemmende Zusatzstoffe wie beispielsweise synthetisch hergestellte Wirkstoffe und /oder Extrakte und /oder Wirkstoffe aus Heilpflanzen, insbesondere Bisobolol und Panthenol können selbstverständlich ebenso zugesetzt werden. Des weiteren können Farbstoffe beispielsweise gelbes und/oder rotes Eisenoxid und /oder Titandioxid zur farblichen Anpassung hinzugegeben werden.

Als Emulgatoren lassen sich anionische, kationische oder neutrale Tenside, beispielsweise Alkaliseifen, Metallseifen, Aminseifen, sulfurierte und sulfonierte Verbindungen, Invertseifen, hohe Fettalkohole, Partialfettsäureester des Sorbitants und Polyoxyethylensorbitans, z.B. Lanette-Typen, Wollwachs, Lanolin oder andere synthetische Produkte zur Herstellung der Öl/Wasser- und /oder Wasser/Öl-Emulsionen einsetzen.

Als Lipide in Form fett- und/oder öl- und/oder wachsartiger Komponenten zur Herstellung der Salben, Cremes oder Emulsionen können Vaseline, natürliche oder synthetische Wachse, Fettsäuren, Fettalkohole, Fettsäureester, z.B. als Mono-, Di- oder Triglyceride, Paraffinöl oder vegetabilische Öle, gehärtetes Rizinusöl oder Kokosöl, Schweinefett, synthetische Fette, z.B. suf Caprryl-, Caprin-, Laurin- und Stearinsäurebasis wie z.B. Softisan® oder Triglyceridgemische wie Miglyol® eingesetzt werden.

Zur Einstellung des pH-Wertes können beispielsweise osmotisch wirksame Säuren und Laugen, z.B. Salzsäure, Citronensäure, Natronlauge, Kalilauge, Natriumhydrogencarbonat, ferner Puffersysteme, wie z.B. Citrat, Phosphat, Tris-Puffer oder Triethanolamin verwendet werden.

Zur Erhöhung der Stabilität können noch Konservierungsmittel, wie beispielsweise Methyl- oder Propylbenzoat (Parabene) oder Sorbinsäure hinzugesetzt werden.

Als weitere topisch applizierbare Formen lassen sich Pasten, Puder oder Lösungen erwähnen. Die Pasten enthalten als konsistenzgebende Grundlagen oft hydrophobe und hydrophile Hilfsstoffe, bevorzugt jedoch hydrophobe Hilfsstoffe mit sehr hohem Feststoffanteil. Die Puder oder topisch applizierbare Pulver können zur Erhöhung der Dispersität sowie des Fließ- und Gleitvermögens sowie zur Verhinderung von Agglomeraten, z.B. Stärkearten, wie Weizen- oder Reisstärke, flammendisperses Siliziumdioxid oder Kieselerden, die auch als Verdünnungsmittel dienen, enthalten.

Die jeweils geeigneten Arzneiformen lassen sich in Einklang mit dem Fachmann bekannten Rezepturvorschriften und Verfahrensweisen auf der Basis pharmazeutisch-physikalischer Grundlagen herstellen.

Das erfindungsgemäße Arzneimittel enthält bevorzugt ca. 35 % (w/w) Isopropylmyristat, ca. 15 % (w/w) mindestens eines Catechins, ca. 24,5 % (w/w) Petrolatum, ca. 20 % (w/w) Wachs, ca. 5 % (w/w) Propylenglykolmonostearat oder Propylenglykolmonopalmitostearat und ca. 0,5 % (w/w) Oleylalkohol.

Das Arzneimittel der vorliegenden Erfindung und /oder eines pharmazeutischen Metaboliten findet seine Anwendung in der Behandlung von viralen Hauterkrankungen und/oder Tumorerkrankungen.

Unter dem Begriff pharmazeutischer Metabolit sind eine oder mehrere Verbindungen zu verstehen, die durch den biologischen Stoffwechsel während der Anwendung entstehen. Es kann sich hierbei um Zwischenprodukte während des intermediären Stoffwechsels oder um Endprodukte des Stoffwechsels handeln. Bevorzugt handelt es sich hierbei um Stoffwechselprodukte, die durch Auftragen auf die Haut und/oder Schleimhaut entstehen, insbesondere Hydrolyseprodukte der Verbindung mit der allgemeinen Formel (I). Denkbare Hydrolyseprodukte können beispielsweise von Rest A und/oder Rest B abgeleitet sein.

Unter viralen Hauterkrankungen werden Hauterkrankungen verstanden, die von Viren ausgelöst bzw. verursacht und/oder mit Virusinfektionen assoziiert sind. Hierzu zählen beispielsweise Hauterkrankungen wie Warzen, Genitalwarzen, durch Papilloma Viren verursachte benigne Tumoren der Haut und/oder Schleimhaut, zum Beispiel Verrucae plantares, Verrucae vulgares, Verrucae planae juveniles, Epidermodysplasia verrucifomis, Condylomata acuminata, Condylomata plana, bowenoide Papulose, Papillome an Larynx und Mundschleimhaut, fokale epitheliale Hyperplasie, Lippenherpes, Kaposi-Sarcom, Windpocken oder Gürtelrose.

Diese viralen Hauterkrankungen und/oder Tumorerkrankungen werden von mindestens einem Papilloma Virus bzw. Viren, insbesondere humane Papilloma Viren, wie zum Beispiel HPV 1, 2, 3, 4, 5, 6, 8, 9, 11, 12 , 13, 14, 15, 16,17, 18, 19-29, 31, 32, 34, 36-38, 46-50, 56, 58, von mindestens einem Herpes Virus bzw. Herpes Viren, wie zum Beispiel Herpes Simplex Virus 1, Herpes Simplex Virus 2, Varicella Zoster Virus oder humanes Herpes Virus wie 1, 2, 3, 4, 7, 8 hervorgerufen.

Die Figuren und die folgenden Beispiele sollen die Erfindung näher erläutern, ohne sie zu beschränken. Die Fachwelt kann im Rahmen des üblichen Könnens die Erfindung entsprechend modifizieren, ohne vom Schutzumfang abzuweichen.

### Beispiele

### Klinische Studie zum Vergleich von einer Salbe und einer Creme enthaltend Isopropylmyristat

In einer multizentrischen klinischen Studie, die an insgesamt 30 verschiedenen Zentren in Deutschland und Rußland durchgeführt wurde, nahmen 93 Patienten teil (jeweils zur Hälfte Männer und Frauen). Die Studie war randomisiert und doppel-verblindet. In der Studie wurde die klinische Wirksamkeit von zwei verschiedenen Formulierungen von Isopropylmyristat (eine Salbe und eine Creme) bei der Behandlung von äußerlichen Genitalwarzen geprüft.

Die getesteten Formulierungen hatten die folgende Zusammensetzung:

### Creme 1:

| **Substanz** | **Menge (in % w/w)** |
|---|---|
| Cera alba | 6,996 |
| Monomuls | 2,798 |
| Lameform TGI | 5,598 |
| Cetiol V | 6,996 |
| Isopropylmyristat | 13,992 |
| Tocopherol | 0,699 |
| Controx KS | 0,066 |
| Glycerin | 6,996 |
| Disodium EDTA | 0,001 |
| Magnesiumsulfate | 1,399 |
| D-Panthenol | 0,699 |
| purified water | 53,678 |
| Red Iron Oxide* | 0,025 |
| Yellow Iron Oxide* | 0,054 |

| | |
|---|---|
| *Die Farbstoffe wurden zur farblichen Anpassung hinzugegeben. | |

### Salbe 1:

| **Substanz** | **Menge (in % w/w)** |
|---|---|
| White Petrolatum, USP | 34,023 |
| White Wax,NF | 25,000 |
| Isopropyl Myristate, NF | 35,000 |
| Oleyl Alcohol, NF | 0,500 |
| Propylene Glycol Monostearate, NF | 5,000 |
| Red Iron Oxide* | 0,022 |
| Yellow Iron Oxide* | 0,055 |
| Titanium Dioxide, USP* | 0,400 |

| | |
|---|---|
| *Die Farbstoffe wurden zur farblichen Anpassung hinzu gegeben. | |

Die Patienten trugen die topische Studienmedikation dreimal täglich oder bis zur vollständigen Heilung der Genitalwarzen oder für maximal 12 Wochen auf.

Während der Studie wurden die folgenden Daten erhoben:

### Vollständige Heilung (in %)

| | **Creme 1** | **Salbe 1** |
|---|---|---|
| männlich | 39,1 | 42,1 |
| weiblich | 35,0 | 33,3 |

Teilweise Heilung (in %); diese entspricht mindestens 75% Heilung bezogen auf die Gesamtfläche der Genitalwarzen.

| | **Creme 1** | **Salbe 1** |
|---|---|---|
| männlich | 43,5 | 63,2 |
| weiblich | 50,0 | 52,4 |

Die Ergebnisse der Studie zeigen, daß im Vergleich zu Plazebo-Werten aus ähnlichen Studien mit unterschiedlichen Formulierungen, bei denen die spontanen Regression von Genitalwarzen bei ca. 20% bei Frauen und ca. 5% bei Männern liegt (Aldara^{™} (Imiquimod) Cream, 5% Product Monograph, vertrieben durch 3M Pharmaceuticals, Northridge, CA, Beutner KR et al. (1998) J Am Acad Dermatol 38, 230-9, Edwards L et al. (1998) Arch Dermatol 134 (1); 25-30) eine überraschend hohe vollständige Heilung bzw. teilweise Heilung erfolgt.

Dieser therapeutische Effekt wird dem Isopropylmyristat zugeschrieben, für das somit erstmals eine antivirale Wirkung gezeigt werden konnte.

### Klinische Studie zum Vergleich von einer Salbe und einer Creme enthaltend Isopropylmyristat und Polyphenon® E

In einer multizentrischen klinischen Studie, die an insgesamt 30 verschiedenen Zentren in Deutschland und Rußland durchgeführt wurde, nahmen 272 Patienten teil (jeweils zur Hälfte Männer und Frauen). Die Studie war randomisiert und doppel-verblindet. In der Studie wurde die klinische Wirksamkeit von zwei verschiedenen Formulierungen von Isopropylmyristat und Polyphenon® E (eine Salbe und eine Creme) gegen die Formulierungen aus Beispiel 1 enthaltend Isopropylmyristat bei der Behandlung von äußerlichen Genitalwarzen geprüft.

Die getesteten Formulierungen mit Polyphenon® E hatten die folgende Zusammensetzung:

### Creme 2:

| **Substanz** | **Menge (in % w/w)** |
|---|---|
| Polyphenon® E | 10,000 |
| Cera alba | 5,263 |
| Monomuls | 2,105 |
| Lameform TGI | 4,211 |
| Cetiol V | 5,263 |
| Isopropylmyristat | 10,526 |
| Tocopherol | 0,526 |
| Controx KS | 0,050 |
| Glycerin | 5,263 |
| Disodium EDTA | 0,001 |
| Magnesiumsulfate | 1,053 |
| D-Panthenol | 0,526 |
| purified water | 55,213 |

### Salbe 2:

| **Substanz** | **Menge (in % w/w)** |
|---|---|
| Polyphenon® E | 15,000 |
| White Petrolatum, USP | 24,500 |
| White Wax,NF | 20,000 |
| Isopropyl Myristate, NF | 35,000 |
| Oleyl Alcohol, NF | 0,500 |
| Propylene Glycol Monostearate, NF | 5,000 |

Die Patienten trugen die topische Studienmedikation dreimal täglich oder bis zur vollständigen Heilung der Genitalwarzen oder für maximal 12 Wochen auf.

Während der Studie wurden die folgenden Daten erhoben:

### Vollständige Heilung (in %)

| | **Creme 1** | **Creme 2** | **Salbe 1** | **Salbe 2** |
|---|---|---|---|---|
| männlich | 39,1 | 53,9 | 42,1 | 61,0 |
| weiblich | 35,0 | 39,5 | 33,3 | 56,8 |

### Teilweise Heilung (≥75% in %)

| | **Creme** | **Creme 2** | **Salbe 1** | **Salbe 2** |
|---|---|---|---|---|
| männlich | 43,5 | 64,2 | 63,2 | 80,5 |
| weiblich | 50,0 | 47,4 | 52,4 | 81,1 |

Die Auswertung der Studie zeigt im Vergleich, daß zwischen Salbe 1 und Creme 1 auf der einen Seite sowie Salbe 2 und Creme 2 auf der anderen Seite die Kombination von Isopropylmyristat und Polyphenon® E zu einer überraschenden Steigerung der Wirksamkeit des Arzneimittels führt.

Vergleicht man nun die Wirksamkeit von Salbe 2 mit der Creme 2, so wird deutlich, daß die Salbe 2 deutlich wirksamer ist als die Creme 2. Dies deutet auf einen synergistischen Effekt zwischen dem Polyphenon® E und dem Isopropylmyristat in der hydrophoben Salbe hin.

Durch diese synergistische Wirkung können die einzelnen Wirkstoffe in der Formulierung in wesentlich geringeren Mengen eingesetzt werden, als die entsprechenden Einzelkomponenten um die selbe Wirkung zu erzielen. Somit hat der Einsatz dieser synergistischen Formulierung nicht nur Vorteile bezüglich der Wirkung, sondern auch bezüglich der Herstellungskosten für diese Formulierung, was sich wiederum positiv auf die Behandlungskosten des Patienten auswirken kann.

Die folgenden Seiten 20 bis 25 enthalten näher definierte Ausführungsformen.
1. Arzneimittel enthaltend eine Verbindung der allgemeinen Formel (I) als pharmazeutisch aktiven Wirkstoff,

   A-B (I)

   **dadurch gekennzeichnet, dass** A ein Rest der allgemeinen Formel (II) 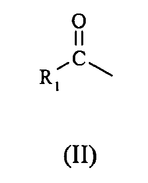 und B ein Rest der allgemeinen Formel (III)

   -O-R₂ (III)

   bedeutet, und
   R₁ unabhängig voneinander ein unverzweigter oder verzweigter, gesättigter, einfach oder mehrfach ungesättigter, gegebenenfalls substituierter C₁₁-C₂₁ Alkyl-, Alkylen- oder Alkinylrest, vorzugsweise ein C₁₁-C₁₅- Alkyl-, Alkylen- oder Alkinylrest, insbesondere ein C₁₁-C₁₃ Alkyl-, Alkylen- oder Alkinylrest, vor allem ein C₁₃-Alkylrest bedeutet, und
   R₂ unabhängig voneinander ein unverzweigter oder verzweigter C₁-C₈ Alkyl Alkylen- oder Alkinylrest, vorzugsweise ein C₁-C₆- Alkyl-, Alkylen- oder Alkinylrest, insbesondere ein C₂-C₄ Alkyl-, Alkylen- oder Alkinylrest, vor allem ein C₃-Alkylrest, ein -[CH₂-(CH₂)ₘ-O]ₙ-H Rest mit n = 1 bis 10, vorzugsweise n = 1 bis 5, m = 1 bis 5, vorzugsweise m = 1 bis 3,
   einen -CH₂-[CH-(OH)]ₚ-[CH₂-(R₃)]-Rest, wobei R₃ unabhängig voneinander ein Wasserstoff oder ein Hydroxylrest, p = 1 bis 7, vorzugsweise p = 1 bis 4, ein Pentose-Rest oder ein Hexose-Rest bedeutet.
2. Arzneimittel nach Nummer 1, **dadurch gekennzeichnet, dass** unabhängig voneinander der Rest R₁ und/oder der Rest R₂ mit einem Halogen, vorzugsweise Fluor und/oder Chlor, einem unverzweigten oder verzweigten C₁-C₆- Alkyl-, Alkylen- oder Alkinylrest, vorzugsweise einem C₁-C₃- Alkyl-, Alkylen- oder Alkinylrest, insbesondere einem Methylrest substituiert ist.
3. Arzneimittel nach Nummer 1 oder 2, **dadurch gekennzeichnet, dass** der Rest A abgeleitet ist von Capronsäure, Caprylsäure, Caprinsäure, Laurinsäure, Myristinsäure, Palmitinsäure, Stearinsäure, Ölsäure, Linolsäure, Linolensäure und/oder Arachidonsäure.
4. Arzneimittel nach Nummer 1 oder 2, **dadurch gekennzeichnet, dass** der Rest B abgeleitet ist von Ethanol, Propanol, Isopropanol, Butanol, Ethylenglykol, Polyethylenglykol, Propylenglykol, Polyproylenglykol, Glycerin, Polyglycerin, Arabit, Adonit, Xylit, Sorbit, Mannit, und/oder Dulcit.
5. Arzneimittel nach mindestens einer der Nummern 1-4, **dadurch gekennzeichnet, dass** die Verbindung (I) Isopropyllaureat, Isopropylmyristat, Isopropylpalmitat, Isopropylstearat, Ethylmyristat, Propylmyristat, Butylmyristat und/oder Ethyloleat ist.
6. Arzneimittel nach mindestens einer der Nummern 1-5, **dadurch gekennzeichnet, dass** das Arzneimittel mindestens 5 % - 75 % (w/w), vorzugsweise mindestens 10 % - 60 % (w/w), insbesondere mindestens 25 % - 55 % (w/w), und vor allem mindestens 35 % - 50 % (w/w) der Verbindung der allgemeinen Formel (I) enthält.
7. Arzneimittel nach mindestens einer der Nummern 1-6, **dadurch gekennzeichnet, dass** das Arzneimittel zusätzlich einen oder mehrere weitere pharmazeutische Wirkstoffe als Kombinationspräparat zur gleichzeitigen, getrennten oder zeitlich abgestuften Anwendung enthält.
8. Arzneimittel nach Nummer 7, **dadurch gekennzeichnet, dass** der zusätzliche pharmazeutische Wirkstoff amphiphil ist.
9. Arzneimittel nach Nummer 7 oder 8, **dadurch gekennzeichnet dass**, der genannte pharmazeutische Wirkstoff mindestens ein Catechin der allgemeinen Formel (IV) enthält, 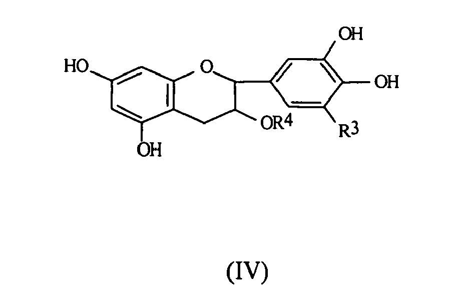 worin,
   R₃ ein -H oder -OH und
   R₄ ein -H oder eine Gruppe der Formel (V) 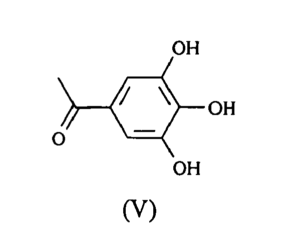 bedeutet.
10. Arzneimittel nach Nummer 9, **dadurch gekennzeichnet, dass** das Catechin ausgewählt ist aus Epicatechin, Epicatechin Gallat, Epigallocatechin, Epigallocatechin Gallat, Gallocatechin und Gallocatechin Gallat, insbesondere (-)-Epicatechin, (-)-Epicatechin Gallat, (-)-Epigallocatechin, (-)-Epigallocatechin Gallat, (+)-Gallocatechin und (-)-Gallocatechin Gallat.
11. Arzneimittel nach Nummer 9 oder 10, **dadurch gekennzeichnet, dass** Catechine in Form eines Gemisches vorliegen, enthaltend 2-20 % (w/w), vorzugsweise 4-15 % (w/w), insbesondere 10-11 % (w/w) (-)-Epicatechin, 2-20 % (w/w), vorzugsweise 5-15 % (w/w), insbesondere 5-7% (w/w) (-)-Epicatechin Gallat, 1-25 % (w/w), vorzugsweise 3-15% (w/w), insbesondere 5-7 % (w/w) (-)-Epigallocatechin, 40-75 % (w/w), vorzugsweise 57-67 % (w/w), insbesondere 61-66 % (w/w) (-)-Epigallocatechin Gallat, 0,05-5 % (w/w), vorzugsweise 0,1-1 % (w/w), insbesondere 0,1-0,6 % (w/w) (+)-Gallocatechin und/oder 0,5-20 % (w/w), vorzugsweise 1-10 % (w/w), insbesondere 1-5 % (w/w) (-)-Gallocatechin Gallat.
12. Arzneimittel nach mindestens einer der Nummern 9-11, **dadurch gekennzeichnet, dass** Catechine in Form eines Gemisches vorliegen, bevorzugt enthaltend 10,8 % (w/w) (-)-Epicatechin, 6,5 % (w/w) (-)-Epicatechin Gallat, 9,2 % (w/w) (-)-Epigallocatechin, 54,8 % (w/w) (-)-Epigallocatechin Gallat und/oder 4,0 % (w/w) (-)-Gallocatechin Gallat.
13. Arzneimittel nach mindestens einer der Nummern 9-12, **dadurch gekennzeichnet, dass** die genannten Catechine aus einem Teeextrakt gewonnen werden.
14. Arzneimittel nach mindestens einer der Nummern 7-13, **dadurch gekennzeichnet, dass** die Formulierung 1-30 % (w/w), vorzugsweise 2-20 % (w/w) und insbesondere 15-18 % (w/w) eines Catechins sowie mindestens 5-90 % (w/w), vorzugsweise mindestens 10-70 % (w/w), insbesondere mindestens 25-60 % (w/w) und vor allem mindestens 35-50 % (w/w) der Verbindung (I) enthält.
15. Arzneimittel nach mindestens einer der Nummern 1-14, **dadurch gekennzeichnet, dass** der pharmazeutische aktive Wirkstoff hydrophob ist.
16. Arzneimittel nach mindestens einer der Nummern 1-15, enthaltend weitere Zusatz- und/oder Hilfsstoffe.
17. Arzneimittel nach Nummer 16, **dadurch gekennzeichnet, dass** die Zusatz- und/oder Hilfsstoffe hydrophob sind, vorzugsweise ausgewählt aus Petrolatum, Wachs, Oleylalkohol, Propylenglykolmonostearat oder Propylenglykolmonopalmitostearat.
18. Arzneimittel enthaltend 35 % (w/w) Isopropylmyristat, 15 % (w/w) mindestens eines Catechins, 24,5 % (w/w) Petrolatum, 20 % (w/w) Wachs, 5 % (w/w) Propyleneglykolmonostearat oder Propylenglykolmonopalmitostearat und 0,5 % (w/w) Oleylalkohol.
19. Verwendung eines Arzneimittels nach mindestens einer der Nummern 1-18 und/oder eines pharmazeutischen Metaboliten davon zur Behandlung von viralen Hauterkrankungen und/oder Tumorerkrankungen.
20. Verwendung eines Arzneimittels nach Nummer 19, **dadurch gekennzeichnet, dass** die viralen Hauterkrankungen und/oder Tumorerkrankungen hervorgerufen werden durch Papilloma Viren, insbesondere durch humane Papilloma Viren, wie zum Beispiel HPV 1, 2, 3, 4, 5, 6, 8, 9, 11, 12, 13, 14, 15, 16, 17, 18, 19-29, 31, 32, 34, 36-38, 46-50, 56, 58, durch Herpes Viren, wie zum Beispiel Herpes Simplex Virus 1, Herpes Simplex Virus 2, Varicella Zoster Virus oder humanes Herpes Virus wie 1, 2, 3, 4, 7, 8.
21. Verwendung eines Arzneimittels nach Nummer 19 oder 20, **dadurch gekennzeichnet, dass** die Hauterkrankungen Warzen, Genitalwarzen, durch Papilloma Viren verursachte benigne Tumoren der Haut und/oder Schleimhaut, zum Beispiel Verrucae plantares, Verrucae vulgares, Verrucae planae juveniles, Epidermodysplasia verrucifomis, Condylomata acuminata, Condylomata plana, bowenoide Papulose, Papillome an Larynx und Mundschleimhaut, fokale epitheliale Hyperplasie, Lippenherpes, Kaposi-Sarcom, Windpocken oder Gürtelrose sind.
22. Verwendung eines Arzneimittels nach mindestens einer der Nummern 1-21, **dadurch gekennzeichnet, dass** das Arzneimittel topisch, insbesondere genital oder vaginal appliziert wird.

## Patentansprüche

1. Arzneimittel enthaltend eine Verbindung der allgemeinen Formel (I)
A-B (I)
und mindestens ein Catechin der allgemeinen Formel (IV), 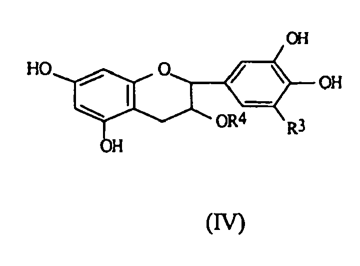 **dadurch gekennzeichnet, dass** A ein Rest der allgemeinen Formel (II) 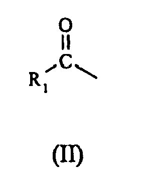 und B ein Rest der allgemeinen Formel (III)
-O-R₂ (III)
bedeutet, und
R₁ unabhängig voneinander ein unverzweigter oder verzweigter, gesättigter, einfach oder mehrfach ungesättigter, gegebenenfalls substituierter C₁₁-C₂₁ Alkyl-, Alkylen- oder Alkinylrest, vorzugsweise ein C₁₁-C₁₅- Alkyl-, Alkylen- oder Alkinylrest, insbesondere ein C₁₁-C₁₃ Alkyl-, Alkylen- oder Alkinylrest, vor allem ein C₁₃-Alkylrest bedeutet, und
R₂ unabhängig voneinander ein unverzweigter oder verzweigter C₁-C₈ Alkyl Alkylen- oder Alkinylrest, vorzugsweise ein C₁-C₆- Alkyl-, Alkylen- oder Alkinylrest, insbesondere ein C₂-C₄ Alkyl-, Alkylen- oder Alkinylrest, vor allem ein C₃-Alkylrest, ein -[CH₂-(CH₂)ₘ-O]ₙ-H Rest mit n = 1 bis 10, vorzugsweise n = 1 bis 5, m = 1 bis 5, vorzugsweise m = 1 bis 3,
einen -CH₂-CH-(OH)]ₚ-[CH₂-(R₃)]-Rest, wobei R₃ unabhängig voneinander ein Wasserstoff oder ein Hydroxylrest, p = 1 bis 7, vorzugsweise p = 1 bis 4, ein Pentose-Rest oder ein Hexose-Rest bedeutet, und
dass in Formel (IV)
R₃ ein -H oder -OH und
R₄ ein -H oder eine Gruppe der Formel (V) 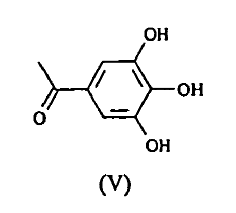 bedeutet,
wobei das Arzneimittel mindestens 5 % - 75 % (w/w), vorzugsweise mindestens 10 % - 60 % (w/w), insbesondere mindestens 25 % - 55 % (w/w), und vor allem mindestens 35 % - 50 % (w/w) der Verbindung der allgemeinen Formel (I) enthält.

2. Arzneimittel nach Anspruch 1, **dadurch gekennzeichnet, dass** unabhängig voneinander der Rest R₁ und/oder der Rest R₂ mit einem Halogen, vorzugsweise Fluor und/oder Chlor, einem unverzweigten oder verzweigten C₁-C₆-Alkyl-, Alkylen- oder Alkinylrest, vorzugsweise einem C₁-C₃- Alkyl-, Alkylen- oder Alkinylrest, insbesondere einem Methylrest substituiert ist.

3. Arzneimittel nach Anspruch 1 oder 2, **dadurch gekennzeichnet, dass** der Rest A abgeleitet ist von von Capronsäure, Caprylsäure, Caprinsäure, Laurinsäure, Myristinsäure, Palmitinsäure, Stearinsäure, Ölsäure, Linolsäure, Linolensäure und/oder Arachidonsäure.

4. Arzneimittel nach Anspruch 1 oder 2, **dadurch gekennzeichnet, dass** der Rest B abgeleitet ist von Ethanol, Propanol, Isopropanol, Butanol, Ethylenglykol, Polyethylenglykol, Propylenglykol, Polyproylenglykol, Glycerin, Polyglycerin, Arabit, Adonit, Xylit, Sorbit, Mannit, und/oder Dulcit.

5. Arzneimittel nach mindestens einem der Ansprüche 1-4, **dadurch gekennzeichnet, dass** die Verbindung (I) Isopropylmyristat ist.

6. Arzneimittel nach mindestens einem der Ansprüche 1-5, **dadurch gekennzeichnet, dass** das Catechin ausgewählt ist aus Epicatechin, Epicatechin Gallat, Epigallocatechin, Epigallocatechin Gallat, Gallocatechin und Gallocatechin Gallat, insbesondere (-)-Epicatechin, (-)-Epicatechin Gallat, (-)-Epigallocatechin, (-)-Epigallocatechin Gallat, (+)-Gallocatechin und (-)-Gallocatechin Gallat.

7. Arzneimittel nach mindesten einem der Ansprüche 1-6, **dadurch gekennzeichnet, dass** Catechine in Form eines Gemisches vorliegen, bevorzugt enthaltend 5,9 % (w/w) (-)-Epicatechin, 12,6 % (w/w) (-)-Epicatechin Gallat, 17,6 % (w/w) (-)-Epigallocatechin, 53,9 % (w/w) (-)-Epigallocatechin Gallat und 1,4 % (w/w) (-)-Gallocatechin.

8. Arzneimittel nach mindestens einem der Ansprüche 1-6, **dadurch gekennzeichnet, dass** Catechine in Form eines Gemisches vorliegen, enthaltend 10,8 % (w/w) (-)-Epicatechin, 6,5 % (w/w) (-)-Epicatechin Gallat, 9,2 % (w/w) (-)-Epigallocatechin, 54,8 % (w/w) (-)-Epigallocatechin Gallat und 4,0 % (w/w) (-)-Gallocatechin Gallat.

9. Arzneimittel nach mindestens einem der Ansprüche 1-8, **dadurch gekennzeichnet, dass** die genannten Catechine aus einem Teeextrakt gewonnen werden.

10. Arzneimittel nach mindestens einem der Ansprüche 1-9, **dadurch gekennzeichnet, dass** die Formulierung 1-30 % (w/w), vorzugsweise 2-20 % (w/w) und insbesondere 15-18 % (w/w) eines Catechins sowie mindestens 5-90 % (w/w), vorzugsweise mindestens 10-70 % (w/w), insbesondere mindestens 25-60 % (w/w) und vor allem mindestens 35-50 % (w/w) der Verbindung (I) enthält.

11. Arzneimittel nach mindestens einem der Ansprüche 1-10, **dadurch gekennzeichnet, dass** der pharmazeutische aktive Wirkstoff hydrophob ist.

12. Arzneimittel nach mindestens einem der Ansprüche 1-11, enthaltend weitere Zusatz- und/oder Hilfsstoffe.

13. Arzneimittel nach Anspruch 12, **dadurch gekennzeichnet, dass** die Zusatz- und/oder Hilfsstoffe hydrophob sind, vorzugsweise ausgewählt aus Petrolatum, Wachs, Oleylalkohol, Propylenglykolmonostearat oder Propylenglykolmonopalmitostearat.

14. Arzneimittel nach einem der Ansprüche 1-13, **dadurch gekennzeichnet, dass** das Arzneimittel 35 % (w/w) Isopropylmyristat, 15 % (w/w) mindestens eines Catechins, 24,5 % (w/w) Petrolatum, 20 % (w/w) Wachs, 5 % (w/w) Propyleneglykolmonostearat oder Propylenglykolmonopalmitostearat und 0,5 % (w/w) Oleylalkohol enthält.

15. Arzneimittel nach mindestens einem der Ansprüche 1-14, **dadurch gekennzeichnet, dass** das Arzneimittel topisch, insbesondere genital oder vaginal appliziert wird.
